Europäisches Patentamt

European Patent Office

Office européen des brevets

(19)

(11) Veröffentlichungsnummer: **0 079 553**
**B1**

(12) **EUROPÄISCHE PATENTSCHRIFT**

(45) Veröffentlichungstag der Patentschrift:
02.10.85

(51) Int. Cl.⁴: **A 61 M 5/16,** B 01 D 35/00

(21) Anmeldenummer: **82110272.0**

(22) Anmeldetag: **08.11.82**

(54) **Mikrofiltrationsgerät zur Filtration von Koageln und Mikroaggregaten von Blut.**

(30) Priorität: **14.11.81 DE 3145320**

(43) Veröffentlichungstag der Anmeldung:
**25.05.83 Patentblatt 83/21**

(45) Bekanntmachung des Hinweises auf die Patenterteilung:
**02.10.85 Patentblatt 85/40**

(84) Benannte Vertragsstaaten:
**AT BE CH FR GB IT LI NL SE**

(56) Entgegenhaltungen:
**EP - A - 0 024 601**
**US - A - 4 056 476**

(73) Patentinhaber: **Biotest-Serum-Institut GmbH,**
**Flughafenstrasse 4, D-6000 Frankfurt-Niederrad (DE)**

(72) Erfinder: **Walker, Wolfram H., Dr. Dipl.-Chem., Leipziger**
**Strasse 12, D-6074 Rödermark (DE)**
Erfinder: **Gänshirt, Karl-Heinz, Dr. Dipl.-Chem.,**
**August-Bebel-Strasse 34, D-6072 Dreieich (DE)**
Erfinder: **Schleussner, Hans, Dr. Dipl.-Chem.,**
**Nansenring 26, D-6000 Frankfurt (DE)**

(74) Vertreter: **Bell, Walter, Dr. et al, BEIL, WOLFF & BEIL**
**Rechtsanwälte Adelonstrasse 58, D-6230 Frankfurt am**
**Main 80 (DE)**

## Beschreibung

Die Erfindung betrifft ein Mikrofiltrationsgerät zur Filtration von Koageln und Mikroaggregaten von Blut.

Bei der Lagerung von Blut enstehen abhängig von verschiedenen Parametern wie Blutqualität, Blutstabilisatorlösung und Lagerungsart Blut Mikrokoagel in der Größenordnung von unter 10 µm bis über 300 µm.

Die Koagel bestehen hauptsächlich aus Thrombozyten, Fibrinogen sowie anderen zellulären und plasmatischen Blutbestandteilen.

Bei der Transfusion von gelagertem Glut und Blutbestandteilen besteht die Gefahr, daß diese Koagel insbesondere bei Massivtransfusion in den Kreislauf eingeschwemmt werden und primär in der Lunge als dem »physiologischen« Filter zurückgehalten werden. Hierbei können diese Koagel zu dem Effekt des sog. »Wet Lung Syndrom« oder der »Transfusionslunge«, d. h. zu einer Verstopfung der Lungenkapillaren beitragen.

Aus diesem Grunde wurden seit Jahren bei Transfusionsbestecken sog. Transfusionsfilter eingesetzt.

Es handelt sich um reine Sieb- oder Maschenfilter mit mittleren Porengrößen von 170—310 µm. So beschreibt DIN 58 360 ein solches Transfusionsbesteck und legt gleichzeitig die Oberfläche mit 10 cm² sowie eine Maschenweite von mind. 310 µm fest.

In den letzten Jahren wurden nun Spezialfilter entwickelt, sog. Mikroaggregat- oder Mikrokoagelfilter, die in der Lage sind, Blutkoagel bis in den Größenbereich von 10 µm aus dem Blut herauszufiltrieren. Bei diesen Mikrokoagelfiltern unterscheidet man zwischen Siebfilter oder Tiefenfilter. Bei Tiefenfiltern beruht der Filtrationseffekt auf einer unselektiven Absorption von Koageln durch eine Schicht mehr oder weniger dicht gepackter Fasern bzw. durch einen porösen Schaum. Siebfilter dagegen arbeiten mit einem Filter definierter Maschenweite. Es gibt auch Kombinationen dieser Filterprinzipien.

An Mikrokoagelfilter werden ganz allgemein von dem Kliniker eine Reihe von Anforderungen gestellt. Hierzu gehört eine hohe Filtereffektivität des Gerätes, eine hohe Filterkapazität, eine hohe Durchflußrate, ein kleines Füllvolumen sowie eine günstige Handhabung des Gerätes.

Außerdem darf ein solches Filter selbst bei Drücken bis zu 400 mm Hg und bei der Verabreichung mehrerer Konserven keinen ungünstigen Einfluß auf die Blutparameter, wie Hämolyse etc., zeigen. Außerdem muß es die Handhabung, d. h. Umstecken auf mehrere Blutkonserven sowie Drucktransfusion ohne Schaden an Filter und Gehäuse überstehen. Es muß andererseits als Einmalgerät möglichst preisgünstig konstruiert sein.

Im DE-U-7 605 700 wird ein Mikrokoagelfilter beschrieben, bei dem das Gerät aus einem Gehäuse besteht, in das bis zu 5 Filter, vorzugsweise mindestens 4 Filter in Form von Sieben, eingebracht werden, die eine Kaskadenanordnung zeigen, d. h., die Siebe werden von dem zu filtrierenden Blut nacheinander durchflossen, wobei das nachfolgende Sieb eine höhere Filterleistung zeigt als das vorausgegangene Sieb. Dadurch kann erreicht werden, daß bei relativ kleiner Filteroberfläche und bei selektiver Beladung der Siebflächen ein günstiger Filtrationseffekt vorliegt.

Das in dem obengenannten Dokument beschriebene Gerät zeigt außerdem eine Tropfkammer, die direkt mit der Filtrationskammer hermetisch verbunden ist. Bei dem gesamten Gerät handelt es sich um ein sterilisierbares Einmalgerät. Die Siebe zeigen bei abnehmender Porengröße eine abnehmende Filterfläche, sind vorzugsweise in Kegelstumpfform ausgebildet und bestehen vorzugsweise aus reinen Siebfiltern, deren kleinste Maschenweite 10 µm beträgt. Das Füllvolumen des Gerätes beträgt mindestens 150 ml. Ein solches Gerät ist z. B. in der Lage, ca. 5—10 ACD-Vollblutkonserven, die ca. 2 Wochen lagerten, innerhalb von 30 Minuten unter Schwerkraftbedingungen zu filtrieren.

Trotz dieser relativ günstigen Eigenschaften erwies sich dieses Gerät häufig als zu groß sowohl im Hinblick auf die Handhabung als auch im Hinblick auf das Füllvolumen. Um unnötigen Blutverlust zu vermeiden, soll das Füllvolumen eines solchen Gerätes so gering wie möglich sein.

Deshalb wurden kleinere Geräte geschaffen. In der DE-U-7 923 865 werden mehrere Modifikationen des vorstehend genannten Gerätes beschrieben, bei dem man nur 2 bis 3 Filterelemente unterschiedlicher Effektivität hintereinandergeschaltet und das ein Füllvolumen von weniger als 150 ml besitzt. Die Kapazität dieses Gerätes erwies sich jedoch als wesentlich geringer und sein Einsatzgebiet beschränkt sich auf die Transfusion von Einzelkonserven und die Filtration von jungen, d. h. nicht gelagerten Vollbluten und Erythrozytenkonzentraten ohne buffy coat (b. c.).

Aus der US-PS 4 056 476 ist ein Mikrofiltrationsgerät zur Filtration von Blut- und Blutbestandteilen bekannt, das eine Filterkammer mit einer Einlauföffnung, einem Filtereinsatz in Form eines Kegelstumpfes, und ein Auslaufteil enthält, wobei Filterkammer, Einlauföffnung und Auslaufteil das Gehäuse des Gerätes bilden. Der Fig. 2 dieses Dokumentes ist auch zu entnehmen, daß der Abstand zwischen dem Filtereinsatz und der Wand der Filterkammer am oberen Ende des Filtereinsatzes größer ist als an seinem unteren Ende. Des weiteren ist eine Stütze für den Filtereinsatz vorgesehen. Dieses Gerät besitzt nur ein einziges Filterelement und weist im Gegensatz zu Geräten, in denen mehrere Filterelemente mit unterschiedlicher Porenweite und unterschiedlicher Oberfläche kaskadenförmig nacheinander angeordnet sind, den Nachteil auf, daß der Filtrationseffekt und die Kapazität im Verhältnis zur Größe des Gerätes äußerst gering ist.

Der Erfindung liegt die Aufgabe zugrunde, ein Gerät bereitzustellen, das den Anforderungen an Mikrokoagelfilter entspricht, d. h. mit dem beispielsweise Massivtransfusionen vorgenommen oder gelagerte alte Vollblute oder Erythrozytenkonzentrate mit b. c. filtriert werden können, jedoch bei ausreichender Effektivität und Kapazität ein kleines Füllvolumen aufweist.

Diese Aufgabe wird überraschenderweise erfindungsgemäß gelöst durch Bereitstellung eines Mikrofiltrationsgerät zur Filtration von Koageln und Mikroaggregaten von Blut und Blutbestandteilen mit einem Füllvolumen von weniger als 100 ml, enthaltend eine Filterkammer, die mit einem Einstichdorn, einem Filtereinsatz mit bis zu vier Filterelementen in der Form eines Kegelstumpfes, wobei diese Filterelemente mit abnehmender Porenweite und abnehmender Oberfläche kaskadenförmig nacheinander angeordnet sind, und einem Auslaufteil versehen ist, sowie eine Tropfkammer, wobei das aus Einstichdorn, Filter- und Tropfkammer bestehende Gehäuse des Gerätes gas- und keimdicht verschlossen ist, dadurch gekennzeichnet, daß die Wand der Filterkammer und der Mantel des Kegelstumpfes unterschiedliche Neigungswinkel gegenüber der Horizontalen aufweisen, wobei an der Basis des Kegelstumpfes die Neigung des Mantels des Kegelstumpfes größer ist als die der Wand der Filterkammer und der Abstand zwischen der Wand und dem Mantel maximal 3 mm beträgt, daß der Filtereinsatz einen in das Filterelement mit der kleinsten Porenweite und der kleinsten Oberfläche hineinragenden und dieses stützenden Abstandshalter aufweist, und jedes der Filterelemente mit mindestens zwei im wesentlichen gegenüberliegenden von der Basis zum Scheitelpunkt des Kegelstumpfes verlaufenden Rippen ausgestattet ist, die maximal 3 mm in das Innere des Kegelstumpfes hineinragen und daß der Abstandshalter so ausgebildet ist, daß er gleichzeitig als Stütze für die Rippen und als Stütze des Filtergewebes des Filterelementes mit der kleinsten Porenweite und der kleinsten Oberfläche dient.

Bestimmte konstruktive Maßnahmen ermöglichen es, mit dem erfindungsgemäßen Gerät trotz des kleinen Füllvolumens von weniger als 100 ml, vorzugsweise 75 ml, die gleiche Filtereffektivität und praktisch die gleiche Kapazität wie bei einem Mikrofiltrationsgerät des gleichen Filterprinzips, jedoch mit einem Füllvolumen von über 150 ml, zu erzielen.

So lassen sich beispielsweise mit dem erfindungsgemäßen Gerät bei gleicher Effektivität wie bei einem Gerät mit einem Füllvolumen von über 150 ml ca. 4—7 ACD Vollblutkonserven, die ca. 2 Wochen lagerten, innerhalb von etwa 15 Minuten unter Schwerkraftbedingungen filtrieren.

Der unterschiedliche Neigungswinkel von Filterkammer und Filtereinsatz bewirkt eine gleichmäßige Beladung des äußersten Filterelementes und verhindert ein Verstopfen des Gerätes auf der Höhe des Einlaufs.

Der Abstand zwischen der Wand der Filterkammer und dem Mantel des Filtereinsatzes von maximal 3 mm ist deshalb wichtig, damit auch größere Aggregate oder Aggregatklumpen, die den Einstichdorn passieren können, das Gerät am Einlauf bzw. über die Länge des Filterelementes (3) nicht verstopfen.

Die längs der kegelstumpfartigen Filterelemente entlanglaufenden Rippen dienen gleichzeitig als Stützstege für das einzelne Filterelement und als Abstandshalter der Filterelemente untereinander und bewirken eine Stabilisierung des Filtergewebes. Ohne diese Rippenanordnung würden sich die Filtergewebe nach Beladung aufeinanderlegen. Hierbei wird die Filterwirksamkeit dieser Siebe fast vollständig aufgehoben.

Die Anzahl der Rippen soll so bemessen sein, daß eine gute Stabilität der Filterelemente erzielt wird, ohne die Filterfläche zu stark zu verringern.

Bei einer bevorzugten Ausführungsform, die in optimaler Weise diesen Forderungen entspricht ist das kleinste Filterelement, d. h. dasjenige mit der kleinsten Porengröße und Filteroberfläche mit zwei Rippen und die übrigen vorzugsweise drei Filterelemente, mit je sechs Rippen ausgestattet. Die Rippen des kleinsten Filterelementes liegen sich vorzugsweise gegenüber und sind mit zwei sich gegenüberliegenden der sechs Rippen des nächstgrößeren Filterelementes deckungsgleich. Die jeweils sechs Rippen der übrigen Filterelemente sind ebenfalls deckungsgleich. Die Tiefe der Rippen von maximal 3 mm ist deshalb wesentlich, weil unter der gegebenen Anordnung sich die Filtersiebe auch bei Massiv- und Drucktransfusion nicht berühren bzw. aufeinanderlegen können und dadurch die Filterwirksamkeit nicht verloren geht.

Die zwei Rippen des letzten, kleinsten Filterelements werden durch einen Abstandshalter, der in den Filtereinsatz hineinragt, fixiert. Dieser Abstandshalter dient gleichzeitig der Verringerung des Füllvolumens und verhindert das Kollabieren der Rippen und des Siebgewebes. Er ist jedoch so gestaltet, daß er die freie Filteroberfläche des kleinsten Filters praktisch nicht beeinträchtigt.

Für die gesamte Kombination werden synthetische blutverträgliche Materialien, wie Polyamide, Polyolefine oder Polyester verwendet. In einer bevorzugten Ausführungsform bestehen Siebkombination und Abstandshalter aus Polyamid, während die Filterkammer aus Polyester, beispielsweise Polyethylentherephtalat besteht.

Als Filterelemente liegen vorteilhafterweise Siebfilter vor, die vorteilhafterweise stehend angeordnet sind. Bei einer bevorzugten Filterkombination liegen vier Filterelemente mit Siebfiltern einer mittleren Maschenweite von 200/50/20/10 $\mu$m vor. Eine zweckmäßige Sieboberflächenkombination ist beispielsweise 69/59/46/33 cm$^2$.

Das hermetische Verschließen oder Verbinden von Filterkammer, Filtereinsatz und Tropfkammer kann durch Verschweißen, Verkleben oder Umspritzen erfolgen.

Ein Ausführungsbeispiel der Erfindung ist in den Zeichnungen dargestellt und wird im folgenden näher beschrieben.

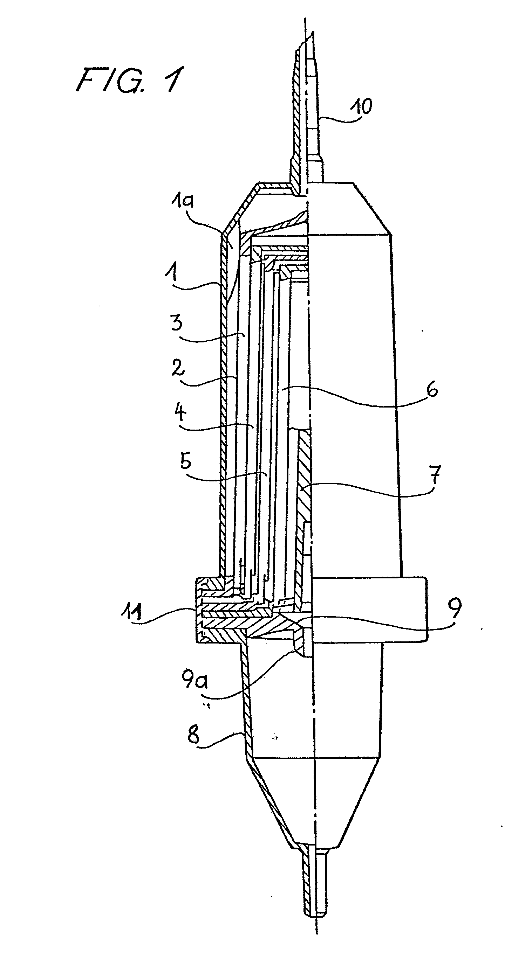

Fig. 1 zeigt einen Aufriß mit $1/2$ Längsschnitt einer bevorzugten Ausführungsform des kompletten Mikrofiltrationsgerätes. Es besteht aus Filterkammer 1 mit Einstichdorn 10 und nach innen ragenden Zungen 1a zum Fixieren des Filtereinsatzes mit Filterelementen 3, 4, 5, 6, die kegelstumpfartig ausgebildet und kaskadenartig stehend angeordnet sind, und Abstandshalter 7. Wie aus der Fig. ersichtlich ist, ist die Neigung des Filtereinsatzes größer als die Neigung der Filterkammer. Unter der Basis des letzten Filterelementes 6 befindet sich ein Auslaufteil 9 in Form einer Platte mit einer mittigen Auslauföffnung 9a in Form eines Auslaufstutzens, der sich die Tropfkammer 8 anschließt. Filterkammer 1, Filtereinsatz 2, Auslaufteil 9 und Tropfkammer 8 werden durch eine Ummantelung 11 nach außen hin hermetisch abgeschlossen.

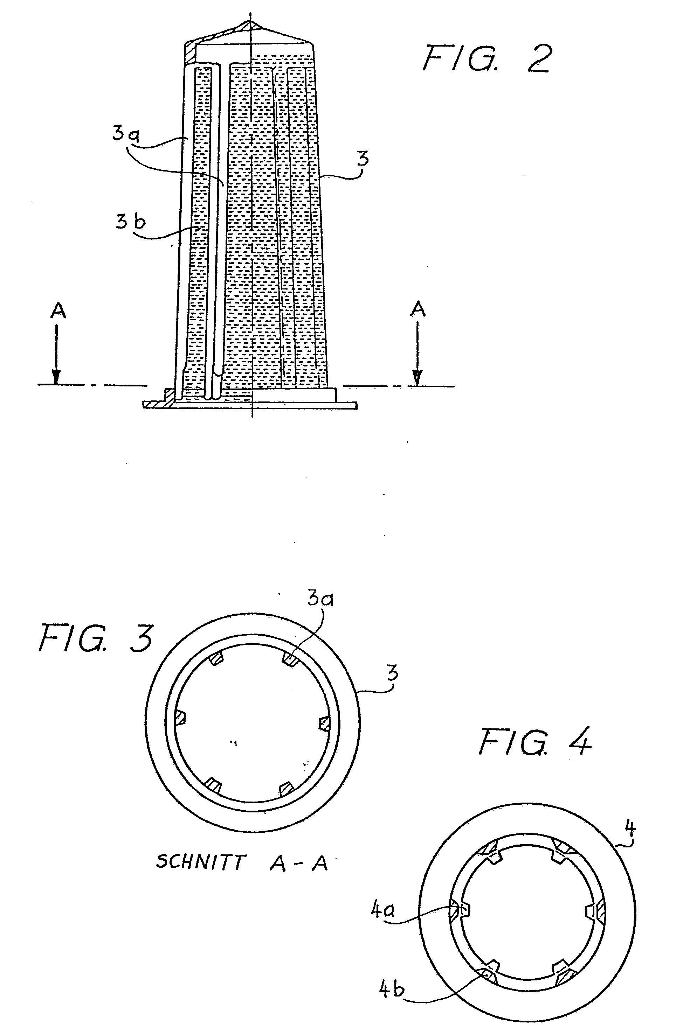

Fig. 2 zeigt einen Aufriß mit $1/2$ Längsschnitt eines Filterelementes 3. Dabei erkennt man Rippen 3a und Siebmaterial 3b.

Fig. 3 ist ein Schnitt entlang der Linie A-A der Fig. 2. Hier erkennt man Filterelement 3 und sechs Rippen 3a.

Fig. 4 zeigt einen Schnitt durch ein Filterelement 4, was dem Schnitt A-A des Filterelements 3 entspricht, mit sechs Rippen 4a und sechs Aussparungen 4b für die Rippen 3a des Filterelements 3. Ein Schnitt durch ein Filterelement 5 würde der Fig. 4 entsprechen, wobei lediglich der Innendurchmesser des Siebkörpers entsprechend kleiner wäre.

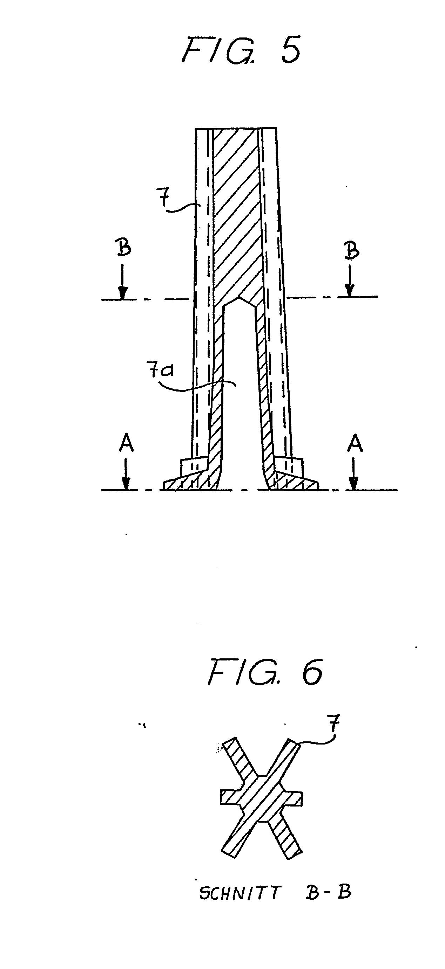

Fig. 5 zeigt einen Längsschnitt durch einen Abstandshalter 7 mit einem Hohlraum 7a.

Fig. 6 zeigt einen Schnitt entlang der Linie B-B der Fig. 5 des Abstandshalters 7, während

Fig. 7 einen Schnitt entlang der Linie A-A der Fig. 5 des Abstandshalters 7 mit Hohlraum 7a zeigt.

Wie der Fig. zu entnehmen ist, dienen die Rippen 7b dazu, das Filtergewebe des Filterelementes 6 zu stützen. Die beiden Rippen 7c dienen dazu, die beiden Rippen 6a des kleinsten Filterelementes 6 zu stützen. Die Höhe des Abstandshalters 7 ist vorzugsweise so ausgelegt, daß sie der halben Höhe des Filterelements 6 entspricht.

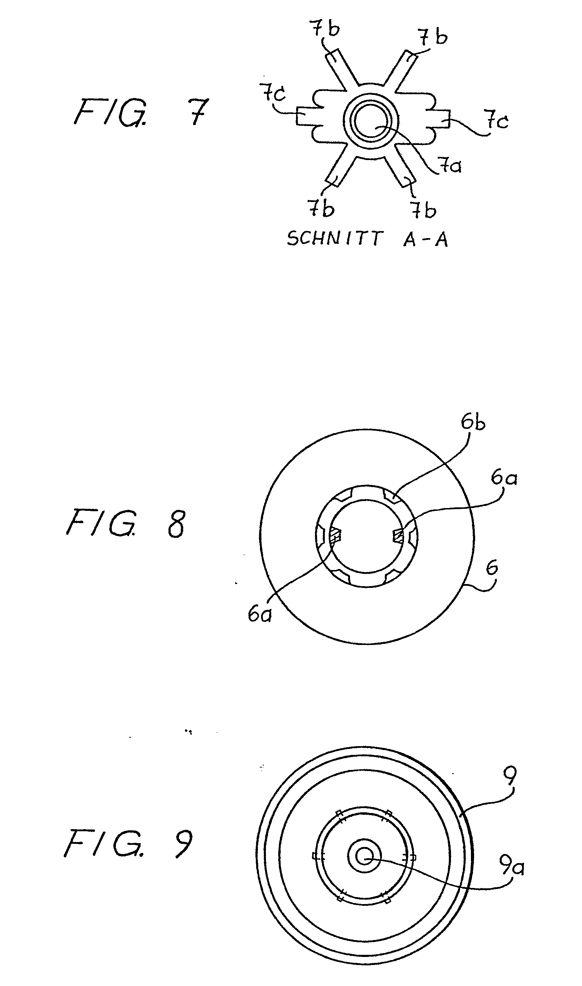

Fig. 8 zeigt einen Schnitt durch ein Filterelement 6, bei dem die zwei Rippen 6a und sechs Aussparungen 6b zu erkennen sind, in die die sechs Rippen des Filterelementes 5 einrasten können.

Fig. 9 zeigt eine Draufsicht auf das Auslaufteil 9 mit Auslauföffnung 9a.

## Patentansprüche

1. Mikrofiltrationsgerät zur Filtration von Koageln und Mikroaggregaten von Blut und Blutbestandteilen mit einem Füllvolumen von weniger als 100 ml, enthaltend eine Filterkammer (1), die mit einem Einstichdorn (10), einem Filtereinsatz (2) mit bis zu vier Filterelementen (3, 4, 5, 6) in der Form eines Kegelstumpfes, wobei diese Filterelemente mit abnehmender Porenweite und abnehmender Oberfläche kaskadenförmig nacheinander angeordnet sind, und einem Auslaufteil (9) versehen ist, sowie eine Tropfkammer (8), wobei das aus Einstichdorn, Filter- und Tropfkammer bestehende Gehäuse des Gerätes gas- und keimdicht verschlossen ist, dadurch gekennzeichnet, daß die Wand der Filterkammer (1) und der Mantel des Kegelstumpfes unterschiedliche Neigungswinkel gegenüber der Horizontalen aufweisen, wobei an der Basis des Kegelstumpfes die Neigung des Mantels des Kegelstumpfes größer ist als die der Wand der Filterkammer und der Abstand zwischen der Wand und dem Mantel maximal 3 mm beträgt, daß der Filtereinsatz (2) einen in das Filterelement (6) mit der kleinsten Porenweite und der kleinsten Oberfläche hineinragenden und dieses stützenden Abstandshalter (7) aufweist, und jedes der Filterelemente (3, 4, 5, 6) mit mindestens zwei im wesentlichen gegenüberliegenden von der Basis zum Scheitelpunkt des Kegelstumpfes verlaufenden Rippen (3a, 4a, 5a, 6a) ausgestattet ist, die maximal 3 mm in das Innere des Kegelstumpfes hineinragen und daß der Abstandshalter (7) so ausgebildet ist, daß er gleichzeitig als Stütze für die Rippen (6a) und als Stütze des Filtergewebes des Filterelementes (6) mit der kleinsten Porenweite und der kleinsten Oberfläche dient.

2. Mikrofiltrationsgerät nach Anspruch 1, dadurch gekennzeichnet, daß das Filterelement (6) mit der kleinsten Porenweite und der kleinsten Oberfläche mit zwei Rippen (6a) und die übrigen Filterelemente (3, 4, 5) mit je sechs Rippen (3a, 4a, 5a) ausgestattet sind, wobei die beiden Rippen des kleinsten Filterelementes mit zwei sich gegenüberliegenden Rippen des nächsten Filterelementes deckungsgleich und die jeweils sechs Rippen der übrigen Filterelemente untereinander deckungsgleich sind.

3. Mikrofiltrationsgerät, nach einem der vorstehenden Ansprüche, dadurch gekennzeichnet, daß die Filterkammer (1) aus Polyester besteht.

4. Mikrofiltrationsgerät, nach einem der vorstehenden Ansprüche, dadurch gekennzeichnet, daß die Filterelemente (3, 4, 5, 6) vier stehend angeordnete Siebfilter mit Porenweiten von 200, 50, 20 und 10 μm sind.

## Claims

1. Microfiltration device for the filtration of coagula and microaggregates from blood and blood components having a capacity of less than 100 ml, containing a filter chamber (1) provided with an attaching chuck (10), a filter cone (2) with up to four truncoconical filter elements (3, 4, 5, 6) which are disposed in cascade one after the other with diminishing pore size and diminishing surface area, a drip chamber (8) and a tailpiece (9), while the casing, consisting of (10), (1) and (8) of the device, is closed in a gas-tight and germ-tight manner, characterized in that filter chamber (1) and filter cone (2) have different angles of inclination, the inclination of the filter cone from the inlet to the outlet being greater than the inclination of the filter chamber, and the distance between filter cone and filter chamber amounting to no more than 3 mm, that filter cone (2) has a spacer (7) extending into the filter element with the smallest pore size and the smallest surface area and supporting same, and each of the filter elements (3, 4, 5, 6) is equipped with at least two substantially opposite ribs (3a, 4a, 5a, 6a) running from the base to the apex of the truncated cone, which extend no more than 3 mm into the interior of the truncated cone, and the space (7) is so constructed that it serves simultaneously as support for the ribs (6a) and as support of the filter fabric of the filter element (6).

2. Microfiltration device of claim 1, characterized in that the filter element (6) with the smallest pore size and the smallest surface area is equipped with to ribs (6a) and the rest of the filter elements (3, 4, 5) are equipped each with six ribs (3a, 4a, 5a), the two ribs of the smallest filter element coinciding with two opposite ribs of the next filter element, and the six ribs of each of the rest of the filter elements coinciding with one another.

3. Microfiltration device of either of the above claims, characterized in that the filter chamber (1) consists of polyester.

4. Microfiltration device of any of the above claims, characterized in that the filter elements (3, 4, 5, 6) are four uprightly disposed sieve filters with pore sizes of 200, 50, 20 and 10 micrometers.

## Revendications

1. Appareil de microfiltration pour la filtration de coagulats et de microagrégats de sang et de composants du sang avec un volume de remplissage inférieur à 100 ml, contenant une chambre de filtration (1) munie d'une aiguille à piqûres (10), d'une cartouche de filtration (2) ayant jusqu'à 4 éléments de filtration (3, 4, 5, 6) sous la forme d'un tronc de cône, ces éléments de filtration étant disposés en cascade par ordre décroissant de diamètre de pores et de surface, et d'une pièce d'écoulement (9), ainsi qu'une chambre de versement goutte à goutte (8), l'enveloppe de l'appareil constituée de l'aiguille à piqûres, de la chambre de filtration et de la chambre de versement goutte à goutte, étant scellée de manière étanche aux gaz et aux germes, caractérisé en ce que la paroi de la chambre de filtration (1) et de l'enveloppe du tronc de cône présentent des angles d'inclinaison différents par rapport à l'horizontale, où à la base du tronc de cône l'inclinaison de l'enveloppe du tronc de cône est plus grande que celle de la paroi de la chambre de filtration et l'intervalle entre la paroi et l'enveloppe s'élève au maximum à 3 mm, en ce que la cartouche de filtration (2) présente un écarteur (7) pénétrant dans l'élément de filtration (6) ayant le plus petit diamètre des pores et la plus faible surface et le soutenant, et en ce que chacun des éléments de filtration (3, 4, 5, 6) est muni d'au moins deux rebords (3a, 4a, 5a, 6a) essentiellement opposés courant de la base au sommet du tronc de cône, qui pénètrent au maximum de 3 mm dans l'intérieur du tronc de cône et en ce que l'écarteur (7) est formé de manière à servir simultanément d'appui pour les rebords (6a) et d'appui du tissu filtrant de l'élément de filtration (6) ayant le plus petit diamètre de pores et la plus petite surface.

2. Appareil de microfiltration selon la revendication 1, caractérisé en ce que l'élément de filtration (6) ayant le plus petit diamètre de pores et la plus petite surface est muni de deux rebords (6a) et les autres éléments de filtration (3, 4, 5) de chacun six rebords (3a, 4a, 5a), où les deux rebords du plus petit élément de filtration sont superposables à deux rebords opposés de l'élément de filtration suivant et où les six rebords respectifs des autres éléments de filtration sont superposables les uns aux autres.

3. Appareil de microfiltration selon l'une des revendications précédentes, caractérisé en ce que la chambre de filtration (1) se compose de polyester.

4. Appareil de microfiltration selon l'une des revendications précédentes, caractérisé en ce que les éléments de filtration (3, 4, 5, 6) sont quatre filtretamis disposés debout ayant des diamètres de pores de 200, 50, 20 et 10 µm.

FIG. 1

FIG. 2

3a

3b

3

A

A

FIG. 3

3a

3

SCHNITT  A - A

FIG. 4

4

4a

4b

9

## FIG. 5

7

7a

B   B

A   A

## FIG. 6

7

SCHNITT B - B

11

FIG. 7

SCHNITT A-A

FIG. 8

FIG. 9